# EUROPÄISCHE PATENTSCHRIFT

(11) **EP 2 257 254 B1**
(45) Veröffentlichungstag und Bekanntmachung des Hinweises auf die Patenterteilung: **04.04.2012**
(21) Anmeldenummer: 09714453.9
(22) Anmeldetag: 20.02.2009
(51) Int. Cl.: A61F 13/02, B32B 15/08, A61N 1/00, A61F 13/15

(54) **PFLASTER**
PATCH
PANSEMENT

(30) Priorität: 28.02.2008 DE 102008011566; 01.08.2008 DE 102008035848
(43) Veröffentlichungstag der Anmeldung: 08.12.2010
(73) Patentinhaber: Barth, Steffen, 87538 Obermaiselstein (DE)
(72) Erfinder: Barth, Steffen, 87538 Obermaiselstein (DE)
(74) Vertreter: Patentanwälte Bressel und Partner
(86) Internationale Anmeldenummer: PCT/EP2009/001365
(87) Internationale Veröffentlichungsnummer: WO 2009/106323

(56) Entgegenhaltungen:
- DE-C1- 3 390 091
- DE-U- 1 798 343
- DE-U1- 8 907 218
- GB-A- 350 384
- GB-A- 926 242
- GB-A- 2 199 501
- US-A- 2 755 800

## Beschreibung

Die Erfindung betrifft ein Pflaster zur Aufbringung auf die Haut für medizinische, kosmetische und/oder orthopädische Zwecke, das ein Trägermaterial aufweist, welches mit einer therapeutisch wirksamen Beschichtung versehen ist.

Pflaster sind in der Medizin und der Kosmetik in verschiedenen Ausprägungen bekannt. Die vorliegende Erfindung befasst sich mit Pflastern zur transdermalen Verabreichung kontrollierter Wirkstoffmengen. Bei solchen Pflastern kann beispielsweise der Wirkstoff in einem Polymermatrixfilm gelöst oder suspendiert sein, durch den der Wirkstoff in die Haut diffundiert (vgl. US 4 839 174).

Die bekannten Pflaster sind nur bedingt zur Behandlung entzündlicher Bereiche geeignet. Insbesondere innere Entzündungen, beispielsweise an Gelenken mit Kapselverletzung oder der so genannte Tennisarm, werden in der Regel durch Stilllegung mittels Bandage und gegebenenfalls mit medikamentöser Unterstützung behandelt. Derartige Behandlungen sind langwierig und können, insbesondere im Fall medikamentöser Behandlung, zu erheblichen Beeinträchtigungen des Körpers führen.

Zur Verbesserung der Behandlung innerer Entzündungen ist aus der DE 103 06 187 A1 ein Pflaster bekannt, welches aus einem Klebestreifen besteht, auf dem eine elektrisch leitfähige Schicht aufgeklebt ist. Die Schicht ist derart auf dem Klebestreifen angeordnet, dass der umlaufende äußere Rand des Klebestreifens unbedeckt ist. Die unbedeckte Klebefläche dient der Fixierung des Pflasters auf der Haut.

Das bekannte Pflaster erfüllt die an es gestellten Anforderungen. Abhängig von der Wahl der elektrisch leitfähigen Schicht ist es jedoch auf einen relativ engen Wirkungsbereich, z.B. entzündliche Vorgänge in einem Gelenk, begrenzt. Auch ist durch die Verwendung eines Klebestreifens, auf den dann die beschichtete Folie aufgebracht ist, eine Dicke des Pflasters hervorgerufen, die bei verschiedenen Anwendungen negative Einflüsse auf den Tragekomfort hat. Dies tritt beispielsweise bei der Behandlung von Kapselentzündungen im Bereich des Fußes, insbesondere im Bereich der Zehen, auf, da die Füße in der Regel im Schuhwerk untergebracht sind.

Bei sportlichen Aktivitäten handelt es sich hierbei meist um recht eng anliegende Schuhe, die dem Träger ein gutes Gefühl für den Kontakt mit dem Boden vermitteln sollen. Darüber hinaus sind beispielsweise Damen-Pumps ebenfalls sehr eng geschnitten. Bei diesen oder ähnlichen Anwendungen sind die bekannten Pflaster nicht ohne Komforteinbußen an den Zehen anbringbar, da durch die Dicke der bekannten Pflaster eine Druckstelle entsteht, die sich genau auf der zu behandelnden Stelle befindet. Durch die Druckstellen sind die Behandlungsergebnisse negativ beeinflusst; im äußersten Fall kann es an den Druckstellen zu Rötungen bis hin zu Hautaufschürfungen kommen. Auch beim Tragen von Kompressionsstrümpfen ergeben sich die genannten Probleme.

Hinzu kommt, dass die bekannten Pflaster das Anziehen der sehr engen Kompressionsstrümpfe behindern bzw. die Pflaster beim Anziehen abgelöst werden.

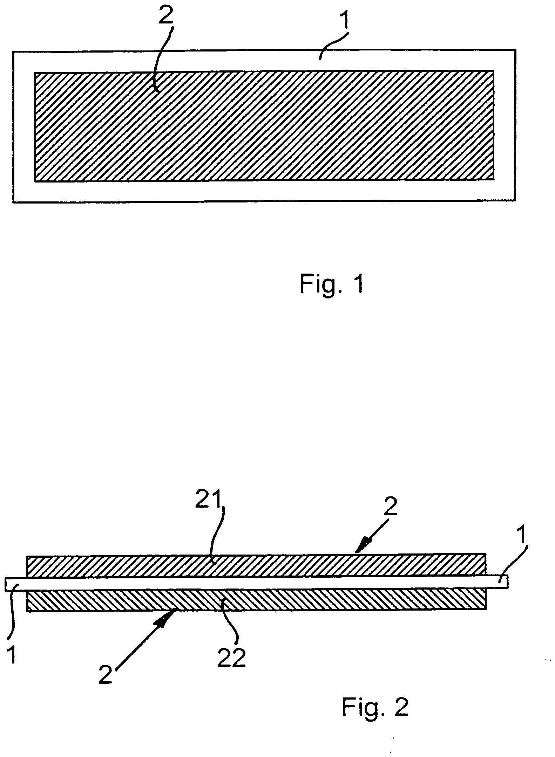

DE 1 798 343 U offenbart einen Verband, insbesondere eine Binde oder ein mit Klebstoff versehendes Pflaster. Der Verband weist zwei durch eine Zwischenlage voneinander getrennte Metallfolien auf, die einem mit Klebstoff versehenden Pflaster zugeordnet sein können. Die eine Folie kann aus einem anderen Werkstoff bestehen als die andere. Als äußere Schicht, die auf den zu behandelnden Körperteil aufgelegt wird, empfiehlt sich eine Folie, die aus einem Edelmetall besteht oder ein solches enthält, beispielsweise Silber, Gold oder dergleichen. Als Ausführungsform wird Kondensatorpapier beschrieben, das aus mehreren, durch Papierlagen getrennte Silberpapieren besteht. Figur 1 zeigt, dass sich eine mit Klebstoff versehene Fläche eines solchen Pflasters umlaufend um das Kondensatorpapier herum erstrecken kann.

US 2,755,800 beschreibt eine klebende Bandage, die eine Metallfolie aufweist, z. B. eine Aluminiumfolie, die auf beiden Seiten auf einem geeigneten flexiblen Pflaster befestigt ist, welches aus Plastik gebildet sein kann. Auf dem Pflaster befindet sich eine Klebstoffbeschichtung als Unterschicht. Randbereiche und die Folie tendieren dazu, eine im Wesentlichen luft- und wasserdichte Abdichtung um die Haut-Verhärtung zu bilden.

Der Erfindung liegt die Aufgabe zugrunde, ein Pflaster zu schaffen, welches die guten Behandlungsergebnisse der aus dem Stand der Technik bekannten Pflaster verbessert und den Anwendungsbereich erweitert. Insbesondere soll das Pflaster abhängig von der biochemischen Situation auf der Haut des Patienten den Behandlungsvorgang selbst steuern können.

GB 926,242 A beschreibt Verbesserungen in Bezug auf reflektierende Schutzfilme. Als Filme werden Olefin-Polymere offenbart, wobei eine reflektierende Metallbeschichtung an zumindest einer Seite vorhanden ist und über die Metallbeschichtung eine Klebstoffschicht überzogen ist, die es ermöglicht, den Film fest auf einer sauberen Oberfläche festzukleben. Der Film ist vorzugsweise nicht dicker als 0,0015 Inch. Als Verfahren zum Beschichten mit Metall werden in Bezug auf Silber, Kupfer oder Gold chemische Reaktionen erwähnt. Die Druckschrift offenbart nicht die Verwendung als Pflaster zur Aufbringung auf die Haut für medizinische, kosmetische und/oder orthopädische Zwecke.

Gemäß einem ersten Aspekt der Erfindung weist das Pflaster eine Folie auf, die aus einem Trägermaterial mit jeweils einer therapeutisch wirksamen Beschichtung an den gegenüberliegenden Seiten des Trägermaterials besteht. Zur Anwendung wird eine der Beschichtungen näher an der Haut des Patienten angeordnet als die andere.

Das Trägermaterial ist z. B. ein natürliches oder künstliches Polymer, vorzugsweise ein Polyester. Insbesondere ist auch eine Polyurethan Matrix als Trägermaterial geeignet. Eine Matrix aus Polymer kann insbesondere Poren, d.h. Durchgangsöffnungen aufweisen, die eine Durchdringung mit Hautschweiß von der einen zur anderen Seite des Trägermaterials ermöglicht.

Experimente haben gezeigt, dass insbesondere bei einer dünnen Trägerschicht von weniger als 100 µm Dicke und insbesondere von weniger als 20 µm Dicke auch die weiter von der Haut entfernt angeordnete Beschichtung zur Therapie (d.h. medizinischen, kosmetischen und/oder orthopädischen Wirkung) beiträgt. Der Beitrag kann auf eine oder mehrere der folgenden Arten geleistet werden:
Bei elektrisch leitfähigen Beschichtungen (zumindest wenn die weiter von der Haut entfernt angeordnete Schicht elektrisch leitend ist) beeinflussen diese durch elektrisch leitenden Kontakt und/oder durch kapazitive Wirkung die biochemischen Prozesse auf, in und unter der Haut. Z.B. bei elektrisch isolierendem Trägermaterial und zwei elektrisch leitfähigen Beschichtungen bildet die Folie einen elektrischen Kondensator. Die Wirkung einer elektrochemischen Batterie wird erzielt, wenn auf den gegenüberliegenden Seiten unterschiedliche Metallbeschichtungen angeordnet sind und in das Trägermaterial Hautschweiß als Elektrolyt eintreten kann, wie es bei Verwendung eines Trägermaterials mit Durchgangsöffnungen der Fall ist.

So kann insbesondere die heilende Wirkung eines Edelmetalls auf der weiter von der Haut entfernt gelegenen Seite (z.B. Gold) entfaltet werden, obwohl das Edelmetall durch das Trägermaterial von der Haut getrennt ist. Gleichzeitig kann z.B. die aus Silber bestehende Beschichtung, die näher an der Haut angeordnet ist (und vorzugsweise unmittelbar mit der Haut oder Absonderungen der Haut, wie Schweiß, in Kontakt ist) ebenfalls die für Silber bekannten heilenden Wirkungen entfalten. Optional kann bei jeder beliebigen Kombination von Beschichtungen zusätzlich ein therapeutisch wirksamer Stoff in dem Trägermaterial enthalten sein oder das Trägermaterial bilden.

Bei jeglicher Art von Beschichtungen können sich die näher gelegene Beschichtung und das Trägermaterial unter dem Einfluss der biochemischen Substanzen auf und in der Haut zumindest stellenweise auflösen, so dass die Beschichtung auf der abgelegenen Seite der Folie sich der Haut nähert oder sogar mit der Haut oder den Substanzen in Kontakt gelangt. Unter "auflösen" wird auch verstanden, dass Beschichtung und Trägermaterial Risse oder Löcher bilden. Zur Auflösung kann es insbesondere dann kommen, wenn der Hautschweiß einen pH-Wert im sauren Bereich hat. Dagegen kann Hautschweiß im basischen Bereich die Trägerfolie unversehrt lassen, z.B. wenn das Trägermaterial ein Polymer (z.B. Polyester) mit weniger als 20 µm Dicke ist. Daher steuert das Pflaster die Therapie abhängig von der biochemischen Situation auf der Haut des Patienten. Auch dient das Pflaster daher als Diagnosehilfe: Wenn der Hautschweiß zur Auflösung von Teilen des Pflasters führt, erlaubt dies den Schluss, dass der Hautschweiß einen pH-Wert im sauren Bereich hat und daher entzündliche Vorgänge vorliegen können. In diesem Fall kann das Pflaster z. B. umgedreht werden, d.h. die Beschichtung der gegenüberliegenden Seite wird näher an der Haut angeordnet.

Das Pflaster kann auf verschiedene Art auf der Haut fixiert werden, wobei die Arten auch miteinander kombiniert werden können. Zumindest eine der Beschichtungen kann stellenweise auf der Oberfläche mit Klebstoff versehen sein. Das Trägermaterial kann selbstklebend sein oder einen Klebstoffauftrag aufweisen, wobei die Beschichtung ausgespart sein kann und/oder das Trägermaterial über den Rand der Beschichtung hinausragen kann. Bevorzugt wird jedoch, dass das Pflaster mit einer zusätzlichen zweiten Trägerschicht kombiniert wird. Auf diese Weise kann das Pflaster selbst sehr dünn ausgeführt werden (siehe unten). Die zweite Trägerschicht ist z.B. eine vollflächig mit Klebstoff versehene Gewebeschicht oder andere Schicht, wie sie üblicherweise bei Tapeverbänden verwendet wird. In diesem Fall kann das Pflaster wahlweise mit der einen oder anderen Beschichtung auf die zweite Trägerschicht aufgeklebt werden, so dass die nicht aufgeklebte Schicht für den Kontakt zur Haut frei bleibt. Die zweite Trägerschicht steht z.B. an den Rändern des so gebildeten Verbandes über, so dass sie auf die Haut geklebt werden kann. Ein solcher oder anderer Verband mit der zweiten Trägerschicht kann fertig hergestellt im Einzelhandel oder der Apotheke angeboten werden. Das Pflaster hat jedoch den Vorteil, dass es auch separat lieferbar ist und daher individuell je nach Anwendungsfall konfigurierbar ist und daher Flächengröße des Pflasters und Art der Fixierung auf der Haut variieren können.

Das Pflaster weist auf den gegenüberliegenden Seiten Beschichtungen auf, die jeweils aus einem elektrisch leitfähigen Material bestehen, nämlich aus Metallen (vorzugsweise, Gold, Silber, Kupfer und/oder Aluminium). Insbesondere wenn durch das Pflaster der Effekt einer elektrochemischen Batterie erzielt wird (siehe oben) können damit neue therapeutische Wirkungen erzielt werden: So werden z. B. die aus der traditionellen chinesischen Medizin bekannten Meridiane nicht nur elektrisch sondern über das durch das als elektrochemische Batterie wirkende Pflaster verbunden, wenn das Pflaster entsprechend auf der Haut angeordnet wird. Um das Eindringen von Hautschweiß in das Trägermaterial zu fördern, kann die an der Hautoberfläche anzuordnende Beschichtung Aussparungen aufweisen, sodass der Hautschweiß unmittelbar in das Trägermaterial eindringen kann. Bei dünner Beschichtung (z. B. 10 nm bis 100 nm dick) kann jedoch Hautschweiß auch durch die Beschichtung hindurch diffundieren.

Die einander gegenüberliegenden Seiten des als Trägerfolie ausgestalteten Trägermaterials sind mit einer insbesondere vollflächig durchgehenden Metallbeschichtung beschichtet. Dadurch wird die von der Haut des Patienten emittierte Wärmestrahlung in besonders hohem Maße zurückreflektiert. Dies gilt auch dann, wenn die Metallbeschichtung auf der näher an der Haut gelegenen Seite sehr dünn ist, z.B. die an anderer Stelle in dieser Beschreibung genannte Dicke aufweist. Selbst wenn noch Wärmestrahlung durch die näher an der Hautoberfläche angeordnete Metallbeschichtung hindurchdringt und auch durch das Trägermaterial hindurchgelangt, wird dieser Strahlungsanteil an der Metallbeschichtung der gegenüberliegenden Seite zurückreflektiert.

Die zweite Beschichtung hat daher den Vorteil, dass trotz der geringen Schichtdicke ein besonders hoher Anteil der Wärmestrahlung auf die Haut zurückreflektiert wird und im Ergebnis die Haut sehr wenig Wärme verliert. Es hat sich gezeigt, dass die Heilungsprozesse in den lebenden Hautschichten und darunter dadurch wesentlich gefördert werden. Dies gilt insbesondere dann, wenn, wie bevorzugt, das Pflaster außerhalb seiner umlaufenden Ränder umlaufend auf die Haut geklebt wird und dadurch kein oder lediglich ein sehr geringer Luftaustausch zwischen der von dem Pflaster abgedeckten Hautoberfläche und der Umgebung stattfinden kann. Ein solcher Luftabschluss wirkt außerdem regulierend auf einen veränderten pH-Wert des Hautschweißes. Insbesondere kann der Hautschweiß nicht oder nur in sehr geringem Maß verdunsten. Dadurch wird einerseits keine Körperwärme für die Verdunstung verbraucht und entsteht andererseits auf der von dem Pflaster abgedeckten Hautoberfläche ein Bereich mit Hautschweiß, der heilend wirkt. Insbesondere bei Silber-oder anderen Metallbeschichtungen auf der näher an der Haut liegenden Seite des Pflasters wird aufgrund der antibakteriellen Wirkung der Hautschweiß gereinigt und kann in gereinigter Form, auch insbesondere ohne Zusatz von Medikamenten, wieder in die oberen Hautschichten eindringen und den Heilungsprozess bzw. den kosmetischen Prozess fördern.

Das Pflaster kann insbesondere auf eine der folgenden Weisen angewendet werden:
- zur Behandlung von Druckstellen, d.h. wenn Druck auf die Haut und die darunter gelegenen Körperbereiche ausgeübt wurde. Die Anwendung ist insbesondere auch dann möglich, wenn sich Blasen gebildet haben. Insbesondere wenn das Pflaster so dünn ausgeführt ist, wie es an anderer Stelle in dieser Beschreibung beschrieben ist, können Kleidung und Schuhe in unveränderter Weise von dem Patienten getragen werden. Das dünne Pflaster trägt nicht wesentlich auf und der Druck auf die Haut wird nicht erhöht. Vielmehr schützen die vorzugsweise als Trägerfolie ausgestaltete Trägerschicht und die Beschichtungen die darunter liegende Hautoberfläche und wirken somit wie eine zusätzliche Hautschicht.
- Besonders geeignet ist das Pflaster zur Anwendung als orthopädisches Pflaster. Insbesondere ist es erstmals möglich, auch ohne Zugabe von Arzneimitteln unter der Haut liegende orthopädische Bereiche heilend zu beeinflussen, indem lediglich ein Pflaster auf die Hautoberfläche aufgebracht wird. Insbesondere durch die oben bereits beschriebenen Prozesse (Wärmeisolierung, Förderung der Schweißbildung, Reinigung des Hautschweißes) wird die Heilung nicht nur in der Haut selbst, sondern auch in den darunter liegenden orthopädischen Körperbereichen (Knochen, Knorpel, Sehnen und Muskulatur) gefördert. Insbesondere können daher Entzündungen in Gelenken, Verstauchungen und Kapselverletzungen mit dem erfindungsgemäßen Pflaster behandelt werden.
- Gemäß der traditionellen chinesischen Medizin (TCM) durch den Körper verlaufende Meridiane können mit Hilfe der auf den gegenüberliegenden Seiten durchgehend und damit elektrisch leitend ausgestalteten Beschichtungen elektrisch verbunden werden. Geeignet ist insbesondere die Anwendung des Pflasters am Fuß oder an der Hand, wo verschiedene Meridiane dicht nebeneinander verlaufen. Es gibt jedoch auch andere Stellen, z.B. am Arm, am Bein oder am Rücken, wo nebeneinander verlaufende Meridiane miteinander elektrisch über die Hautoberfläche verbunden werden können.
- Das Pflaster kann zur Diagnose eingesetzt werden, ob eine Entzündung oder andere Erkrankung vorliegt. Hierzu wird das Pflaster auf die Hautoberfläche der Körperstelle aufgebracht, insbesondere in der oben beschriebenen Weise mit Luftabschluss gegen die Umgebung. Der insbesondere durch die Wärmeisolierung verursachte Hautschweiß kann mit der näher an der Hautoberfläche liegenden Beschichtung wechselwirken. Unter bestimmten, an anderer Stelle in dieser Beschreibung erwähnten Umständen kann der Hautschweiß zusätzlich mit der an der gegenüberliegenden Seite angeordneten zweiten Beschichtung wechselwirken. Durch diese Wechselwirkung oder Wechselwirkungen und etwaig auftretende Beschädigungen des Trägermaterials, die abhängig von der chemischen Zusammensetzung des Hautschweißes sind, kann festgestellt werden, ob der Hautschweiß "normal" ist, d.h. der abgedeckte Körperbereich gesund ist, oder ob Veränderungen gegenüber dem normalen Zustand auf eine Störung oder Erkrankung hinweisen. Die Zusammensetzung des Hautschweißes, die insbesondere an dem pH-Wert der Zusammensetzung erkennbar ist, wirkt insbesondere auf die vorzugsweise als Beschichtungsmaterial verwendeten Metalle ein. Ein wesentlicher Prozess dabei ist die Beeinflussung der normalerweise an der Oberfläche von Metallen auftretenden Oxidschichtbildung. Substanzen in dem Hautschweiß, insbesondere Chlor, verhindern die Oxidschichtbildung. Z.B. Aluminium, das normalerweise eine stabile Schutzschicht aus Aluminiumoxid bildet, kann daher von anderen Substanzen in dem Hautschweiß angegriffen werden, d.h. diese Substanzen können mit dem Aluminium reagieren und daher die Aluminiumbeschichtung zumindest stellenweise auflösen. Umgekehrt kann der Hautschweiß bei Verwendung von Edelmetallen für die Beschichtung der Trägerfolie je nach Zusammensetzung des Hautschweißes zu einer Oxidschichtbildung beitragen, die andernfalls nicht stattfinden würde, da Edelmetalle nicht oder nur in geringem Umfang mit Luftsauerstoff reagieren. Die gebildete Oxidschicht kann auf einfache Weise erkannt werden. Die Diagnosefähigkeit des Pflasters ist besonders hoch, wenn das Pflaster unter Abschluss gegen die Umgebungsluft auf der Hautoberfläche angeordnet wird. Unter "Abschluss" wird auch eine wesentliche Behinderung des Luftaustauschs verstanden, die z.B. dann erreicht ist, wenn wie oben beschrieben der Rand des Pflasters umlaufend mit der Haut verklebt ist.

Weitere Anwendungen des Pflasters werden im Folgenden kurz erwähnt. So lassen sich insbesondere Narben mit wulstartigen Verklebungen von Hautschichten und Verhärtungen durch Aufbringen des Pflasters behandeln. Im Ergebnis wird die Haut insbesondere durch Förderung der Durchblutung wieder geschmeidiger, was sich auch kosmetisch dadurch auswirkt, dass die Narbe weniger auffällt.

Insbesondere durch die wärmeisolierende und daher durchblutungsfördernde Wirkung des Pflasters, aber auch durch andere Prozesse, die durch das Pflaster angeregt werden, wird die Zellteilung in dem von dem Pflaster abgedeckten Körperbereich beschleunigt.

Insbesondere bei einer über die Oberfläche der Trägerfolie durchgehenden elektrisch leitfähigen Beschichtung aus Metall wird der gesamte von dem Pflaster abgedeckte Bereich des Körpers in gleicher Weise beeinflusst. Dies ist z.B. bei Verwendung von Pflastern, deren Trägermaterial ein Gewebe ist oder ein Vlies ist, nicht erzielt. Mit dem erfindungsgemäßen Pflaster tritt aufgrund dieser Gleichbehandlung des abgedeckten Bereichs, in dem sich vorzugsweise nicht nur erkrankte Teilbereiche, sondern auch gesunde Teilbereiche befinden, eine Harmonisierung ein, d.h. gesunde Teilbereiche können auf kranke Teilbereiche einwirken und die Heilung fördern. Diese harmonisierende Wirkung tritt insbesondere dann ein, wenn bei einer gleichmäßig dicken Trägerfolie die einander gegenüberliegenden Seiten jeweils mit einer Metallbeschichtung versehen sind und die wie oben beschrieben an beiden Metallbeschichtungen in Richtung der Haut zurückreflektierten Infrarotstrahlen und die von der Haut abgestrahlten Infrarotstrahlen in gleicher Weise interferieren, unabhängig davon, an welcher Stelle des Pflasters man diese Interferenzprozesse betrachtet. Daher wird entsprechend der vorzugsweise konstanten Dicke der Trägerfolie und entsprechend der Reflexionseigenschaften der Metallbeschichtungen ein bestimmtes Spektrum von Infrarotstrahlung erzeugt, das überall in dem durch das Pflaster abgedeckten Bereich in gleicher Weise auf die Haut und die darunter liegenden Bereiche einwirkt.

Insbesondere durch Aufbringen des Pflasters im Bereich des Brustbeins auf die Hautoberfläche können asthmatische Erscheinungen behandelt werden, insbesondere solche, die durch Kälteeinwirkung entstanden sind.

Durch Aufbringen des Pflasters auf Hautoberflächen, die sich nahe der Atemwege des Körpers befinden (z.B. Nase, Hals oder Brustbeinbereich) können Erkrankungen der Atemwege und damit zusammenhängende Erkrankungen behandelt werden.

Durch Aufbringen des Pflasters auf Hautfalten kann die Auffälligkeit dieser Falten verringert werden. Insbesondere wird die Haut im Bereich der Falten besser durchblutet, dadurch geschmeidiger und erscheint dadurch weniger tief. Insbesondere bei dieser Anwendung, aber auch generell, kann das Pflaster aufgrund der durchblutungsfördernden Wirkung Schlackenstoffe abtransportieren helfen und die Haut jünger erscheinen lassen.

Durch Aufbringen des Pflasters auf Bereiche, unter denen sich Venen und/oder Lymphgefäße befinden, kann der Durchfluss durch diese Gefäße gefördert werden. Oben wurde erwähnt, dass die Beschichtung auf der einen Seite der Trägerfolie z.B. aus Gold besteht und auf der anderen Seite aus Silber besteht. Ähnliche heilende Wirkungen wie mit der Kombination Gold/Silber können jedoch auch dann erzielt werden, wenn statt Silber silberfarbene Stoffe verwendet werden und/oder wenn statt Gold goldfarbene Stoffe verwendet werden. Silberfarbene Stoffe sind insbesondere ähnlich wie Silber schimmernde Metalle, z.B. Aluminium. Ähnlich wie Gold aussehende Metalle, d.h. goldfarbene Metalle, sind z.B. Messing oder eloxiertes Aluminium, das entsprechend gefärbt wurde (so genanntes Goldeloxal).

Nicht zum Umfang der beigefügten Ansprüche gehört ein Pflaster, das nur eine Beschichtung aufweist. Das Pflaster weist gemäß den Ansprüchen vielmehr zwei Beschichtungen auf. In jedem Fall beträgt die Dicke der Folie (Trägermaterial und Beschichtungen) weniger als 100µm, vorzugsweise weniger als 20 µm und liegt z.B. im Bereich von 12-15µm.

Somit ist ein Pflaster geschaffen, das die guten Behandlungsergebnisse der bekannten Pflaster beinhaltet, gleichzeitig jedoch aufgrund der außerordentlich dünnen Ausführung keine Behinderung des Tragekomforts bzw. der Handhabung hervorruft. Insbesondere wird der Tragekomfort beziehungsweise die Anwendbarkeit auch in engem Schuhwerk oder Kompressionsstrümpfen ermöglicht.

Weiterbildungen und Ausgestaltungen der Erfindung sind in den Unteransprüchen angegeben. Ein Ausführungsbeispiel der Erfindung ist in der Zeichnung dargestellt und wird nachfolgend im Einzelnen beschrieben. Es zeigen:
- Fig. 1: die Ansicht eines erfindungsgemäßen Pflasters und
- Fig. 2: die überhöhte Seitenansicht des in Fig. 1 dargestellten Pflasters.

Bei dem als Ausführungsbeispiel gewählten Pflaster handelt es sich um eine metallisierte Polyester-Folie 1. Sie bildet das Trägermaterial für das Pflaster. Die Folie hat z.B. eine Dicke von 10 bis 15 µm, im Ausführungsbeispiel 12 µm. Die Folie kann dehnbar sein. Die Dehnungseigenschaften liegen in Längsrichtung z. B. bei 100 bis 130 %; die Querdehnung liegt z. B. bei etwa 100 %.

Die Folie 1 ist mit einer Beschichtung 2 versehen. Bei der Beschichtung 2 kann es sich um eine Schutzlackierung handeln; vorzugsweise um eine Gold- oder Silber-Schutzlackierung. In Abwandlung kann es sich um eine Pulverbeschichtung handeln, die ebenfalls vorzugsweise aus Silber oder Gold besteht und die mit der Folie 1 verklebt oder auf andere Weise daran befestigt ist. Die Auftragsmenge der Beschichtung beträgt z. B. 0,8 bis 1,2 g/qm. Im Ausführungsbeispiel ist die Beschichtung auf beiden Seiten der Folie 1 aufgebracht, wobei die mit "21" bezeichnete Seite mit einer Beschichtung aus Gold und die mit "22" bezeichnete andere Seite mit einer Beschichtung aus Silber versehen ist. Alternativ zu der Pulverbeschichtung sind das Gold und/oder Silber im Vakuum (insbesondere im Hochvakuum) auf das Trägermaterial aufgedampft worden.

Die Folie 1 kann selbstklebend ausgebildet sein. Der Klebstoff könnte über die gesamte Fläche des Pflasters verbreitet sein, ist jedoch gemäß den Ansprüchen an den umlaufenden Rändern der Folie 1 umlaufend angeordnet. Alternativ weist die Folie keinen außenliegenden Klebstoff auf, sondern wird mit zusätzlichen Mitteln (siehe oben) an der Haut des Patienten fixiert.

Die Folie 1 ist z. B. nicht oder nur in geringem Umfang luftdurchlässig ausgeführt. Dadurch wird nach dem Fixieren der Folie auf die Haut eine Schweißbildung im Bereich des Pflasters gefördert. Die Schweißbildung trägt zu einer erhöhten Leitfähigkeit der metallisierten Folie bei. Durch das Auftreten des Schweißes in Verbindung mit Luft wird aus der Folie Gold beziehungsweise Silber gelöst, weil das im Schweiß vorhandene Chlorid die Ausbildung einer Schutzschicht auf dem Gold verhindert, so dass der Luftsauerstoff in Verbindung mit dem relativ niedrigen pH-Wert der feuchten Haut das Gold beziehungsweise Silber in Lösung bringt. Hierdurch ist die Leitfähigkeit des Pflasters auf der Haut erhöht, wodurch das Heilungsverfahren zusätzlich optimiert ist. Zudem führt das Schwitzen unter dem Pflaster zum Aufweichen der Haut, was den Heilungsprozess beispielsweise bei Narben zusätzlich verbessert. Außerdem werden durch das Schwitzen körperunreine Stoffe ausgeschieden, was zu einer Verbesserung des Stoffwechsels führt. Auch führen die genannten Effekte dazu, dass Hautschweiß in das Trägermaterial eindringen kann und somit auch die Beschichtung an der weiter von der Haut entfernt gelegenen Seite einen therapeutischen Beitrag liefert.

Das erfindungsgemäße Pflaster ist vorzugsweise sehr dünn (z. B. höchstens 20 Mikrometer dick) ausgeführt. Dadurch ist ein Tragen des Pflasters auch in Bereichen möglich, in denen bekannte Pflaster eine Beeinträchtigung des Tragekomforts hervorrufen würden. Dies gilt insbesondere in Schuhen, besonders im Bereich der Zehen, oder bei Skischuhen im Bereich der bekannten Druckstellen am Knöchel, dem Schienbein usw. Das Gleiche gilt auch beim Tragen von Kompressionsstrümpfen, z.B. bei Venen- oder Lymphschwäche. In den genannten Bereichen ist kein Raum zwischen Haut und Schuhwerk bzw. Strumpf, so dass bekannte Pflaster immer "auftragen" und damit der Druck an der jeweiligen Stelle noch weiter erhöht wird. Dies ist bei dem erfindungsgemäßen Pflaster vermieden; aufgrund seiner außerordentlich dünnen Ausbildung ist das Pflaster kaum spürbar.

Aufgrund der dünnen Ausführung des erfindungsgemäßen Pflasters ist auch die Handhabung von Kompressionsstrümpfen wesentlich vereinfacht, da bei diesen üblicherweise bei der Verwendung von herkömmlichen Pflastern ein Ablösen bzw. Abrubbeln beim Überstreifen der Strümpfe erfolgt. Dadurch verlieren die Pflaster ihre beabsichtigte Positionierung, so dass die Wirkung nicht zielgerichtet erfolgen kann. Bei den hauchdünnen Pflastern nach der Erfindung ist dieses Problem vermieden, da sich die Kompressionsstrümpfe ungehindert über das Pflaster streifen lassen; bei der selbstklebenden Ausbildung über die gesamte Fläche des Pflasters sind zudem gute Hafteigenschaften hervorgerufen, die ein Anhaften an der Haut beim Überziehen der Strümpfe ermöglichen. Durch die guten Hafteigenschaften ist zudem ein Ablösen bei längerer Tragedauer vermieden, so dass das erfindungsgemäße Pflaster auch über mehrere Tage problemlos eingesetzt werden kann.

Das Vorsehen der Beschichtung auf beiden Seiten des Pflasters, insbesondere bei Verwendung unterschiedlicher Beschichtungen auf den beiden Seiten des Pflasters, beispielsweise der Beschichtung aus Gold auf der einen und Silber auf der anderen Seite nach dem Ausführungsbeispiel, bietet die Möglichkeit, je nach Anwendungsfall eine Behandlungsart auszuwählen. So wird die mit Silber beschichtete Seite in der Regel näher an der Haut angeordnet, wenn eine Kühlung und entzündungshemmende Wirkung erzielt werden soll, die eine Beruhigung bewirkt und dazu beiträgt, Schwellungen abzubauen. Die goldbeschichtete Seite wird näher an der Haut angeordnet, wenn eine Wärmung an der betroffenen Stelle erforderlich ist, da Gold bekanntermaßen die Durchblutung anregt, was zu einer erhöhten Blutzirkulation führt, die eine wärmende Wirkung auslöst. Es sind somit in dem erfindungsgemäßen Pflaster zwei unterschiedliche Behandlungs- und Anwendungsfälle zusammengefasst, so dass nur ein Pflaster zu bevorraten ist, um beide Fälle behandeln zu können. Die Auswahl erfolgt unmittelbar vor der Verwendung des Pflasters. Dies beinhaltet für den Anwender den Vorteil, bei nur einem mitzuführenden Pflaster zwei Behandlungsfälle abdecken zu können.

Wie oben beschrieben trägt jedoch auch die weiter von der Haut entfernte Beschichtung zur Therapie bei, insbesondere wenn das Trägermaterial sehr dünn ausgeführt ist oder sich im Laufe der Anwendung auflöst.

## Patentansprüche

1. Pflaster zur Aufbringung auf die Haut für medizinische, kosmetische und/oder orthopädische Zwecke, wobei
• das Pflaster eine Folie (1) aufweist, die aus einem Trägermaterial besteht, das auf beiden Seiten mit einer therapeutisch wirksamen Beschichtung (21, 22) versehen ist,
• an den umlaufenden Rändern der Folie (1) und/oder außerhalb der umlaufenden Ränder der Folie (1) umlaufend Klebstoff vorgesehen ist, so dass durch Aufkleben des Pflasters auf die Haut eines Patienten der von der Folie (1) abgedeckte Hautbereich gegen die Luft in der Umgebung abgeschlossen wird,
• es sich bei der Beschichtung (22) auf einer Seite um eine Silber-Beschichtung oder silberfarbene Metallbeschichtung handelt,
• es sich bei der Beschichtung (21) auf der gegenüberliegenden Seite um eine Gold-Beschichtung oder goldfarbene Metallbeschichtung handelt,
• dass die Folie (1) einschließlich beider Beschichtungen (21, 22) eine Dicke von weniger als 100 µm hat.

2. Pflaster nach Anspruch 1, **dadurch gekennzeichnet, dass** die Folie (1) einschließlich beider Beschichtungen (21, 22) eine Dicke von weniger als 20 µm hat.

3. Pflaster nach Anspruch 1 oder 2, **dadurch gekennzeichnet, dass** die Folie (1) dehnbar ist.

4. Pflaster nach einem oder mehreren der vorgenannten Ansprüche, **dadurch gekennzeichnet, dass** die Beschichtung (2) auf einer Seite eine Schutzlackierung ist.

5. Pflaster nach einem oder mehreren der vorgenannten Ansprüche, **dadurch gekennzeichnet, dass** die Beschichtung (2) auf einer Seite eine Pulverbeschichtung ist.

6. Pflaster nach einem oder mehreren der vorgenannten Ansprüche, **dadurch gekennzeichnet, dass** die Beschichtungen (21, 22) auf den gegenüberliegenden Seiten jeweils aus einem elektrisch leitfähigen Material bestehen.

7. Pflaster nach dem vorhergehenden Anspruch, **dadurch gekennzeichnet, dass** zumindest eines der elektrisch leitfähigen Materialien ein Edelmetall ist.

8. Pflaster nach einem oder mehreren der vorgenannten Ansprüche, **dadurch gekennzeichnet, dass** das Trägermaterial aus einem Polymer besteht.

9. Pflaster nach dem vorhergehenden Anspruch, **dadurch gekennzeichnet, dass** das Trägermaterial aus Polyester besteht.

10. Pflaster nach einem oder mehreren der vorgenannten Ansprüche, **dadurch gekennzeichnet, dass** die Beschichtung auf zumindest einer Seite eine Dicke von weniger als 5 µm, vorzugsweise weniger als 500 nm hat.

11. Pflaster nach einem oder mehreren der vorgenannten Ansprüche, **dadurch gekennzeichnet, dass** die Beschichtungen durch Aufdampfen von Silber bzw. Gold im Vakuum auf die Folie (1) erzeugt worden sind.

12. Verfahren zur Herstellung eines Pflasters zur Aufbringung auf die Haut für medizinische, kosmetische und/oder orthopädische Zwecke, insbesondere zur Herstellung des Pflasters nach einem der vorhergehenden Ansprüche, wobei
• eine Folie (1) aus einem Trägermaterial derart auf einer ersten Seite mit einer Gold-Beschichtung (21) oder goldfarbenen Metallbeschichtung versehen wird und
• die Folie (1) derart auf einer gegenübertlegenden, zweiten Seite mit einer Silber-Beschichtung (22) oder silberfarbenen Metallbeschichtung versehen wird, dass die Folie (1) einschließlich beider Beschichtungen (21, 22) eine Dicke von weniger als 100 µm hat,
wobei an den umlaufenden Rändern der Folie (1) und/oder außerhalb der umlaufenden Ränder der Folie (1) umlaufend Klebstoff vorgesehen wird, so dass durch Aufkleben des Pflasters auf die Haut eines Patienten der von der Folie (1) abgedeckte Hautbereich gegen die Luft in der Umgebung abgeschlossen wird.

13. Verfahren nach dem vorhergehenden Anspruch, wobei die Beschichtungen durch Herstellen einer Schutzlackierung auf der Folie (1), Herstellen einer Pulverbeschichtung auf der Folie (1) oder durch Aufdampfen von Silber bzw. Gold im Vakuum auf die Folie (1) erzeugt werden.

## Claims

1. A plaster to be applied to the skin for medical, cosmetic and/or orthopedic purposes, wherein
• the plaster has a foil (1) which comprises a carrier material which has a therapeutically effective coating (21, 22) on both sides (21, 22),
• an adhesive is provided circumferentially on the circumferential edges of the foil (1) and/or outside of the circumferential edges of the foil (1) so that through sticking the plaster to a patient's skin the area of the skin which is covered by the foil (1) is sealed off from the ambient air,
• the coating (22) on one side is a silver coating or a silver-colored metal coating,
• the coating (21) on the opposite side is a gold coating or a gold-colored metal coating,
• the foil (1) including both coatings (21, 22) has a thickness of less than 100 µm.

2. The plaster according to Claim 1, **characterized in that** the foil (1) including both coatings (21, 22) has a thickness of less than 20 µm.

3. The plaster according to Claim 1 or 2, **characterized in that** the foil (1) is stretchable.

4. The plaster according to one or more of the preceding claims, **characterized in that** the coating (2) on one side is a protective painting.

5. The plaster according to one or more of the preceding claims, **characterized in that** the coating (2) on one side is a powder coating.

6. The plaster according to one or more of the preceding claims, **characterized in that** the coatings (21, 22) on the opposing sides each consist of an electrically conductive material.

7. The plaster according to the preceding claim, **characterized in that** at least one of the electrically conductive materials is a noble metal.

8. The plaster according to one or more of the preceding claims, **characterized in that** the carrier material consists of a polymer.

9. The plaster according to the preceding claim, **characterized in that** the carrier material consists of polyester.

10. The plaster according to one or more of the preceding claims, **characterized in that** the coating on at least one side has a thickness of less than 5 µm, preferably less than 500 nm.

11. The plaster according to one or more of the preceding claims, **characterized in that** the coatings were created through vapor depositing of silver and/or gold in the vacuum onto the foil (1).

12. A process for manufacturing a plaster to be applied to the skin for medical, cosmetic and/or orthopedic purposes, in particular for manufacturing a plaster according to any one of the preceding claims, wherein
• a foil (1) made from a carrier material is provided on a first side with a gold coating (21) or a gold-colored metal coating and
• the foil (1) is provided on an opposite, second side with a silver coating (22) or a silver-colored metal coating, both in such a way
that the foil (1) including both coatings (21, 22) has a thickness of less than 100 µm,
wherein an adhesive is provided circumferentially on the circumferential edges of the foil (1) and/or outside of the circumferential edges of the foil (1) so that through sticking the plaster to a patient's skin the area of the skin which is covered by the foil (1) is sealed off from the ambient air.

13. The process according to the preceding claim, wherein the coatings are created through manufacture of a protective painting on the foil (1), manufacture of a powder coating on the foil (1) or through vapor depositing of silver and/or gold in the vacuum onto the foil (1).

## Revendications

1. Pansement adhésif destiné à être appliqué sur la peau à des fins médicales, cosmétiques et/ou orthopédiques, étant donné que
• le pansement adhésif présente une pellicule (1) qui se compose d'un support qui est doté sur les deux côtés d'un revêtement (21, 22) à effet thérapeutique,
• de la colle est prévue circonférentiellement sur les bords circonférentiels de la pellicule (1) et/ou hors des bords circonférentiels de la pellicule (1), de manière à ce que, lorsque le pansement adhésif est collé sur la peau d'un patient, la surface de la peau recouverte par la pellicule (1) soit étanche à l'air ambiant,
• le revêtement (22) sur un côté est un revêtement en argent ou un revêtement métallique argenté,
• le revêtement (21) sur le côté opposé est un revêtement en or ou un revêtement métallique couleur or,
• la pellicule (1), y compris les deux revêtements (21, 22), a une épaisseur de moins de 100 µm.

2. Pansement adhésif selon la revendication 1, **caractérisé en ce que** la pellicule (1), compris les deux revêtements (21, 22), a une épaisseur de moins de 20 µm.

3. Pansement adhésif selon la revendication 1 ou 2, **caractérisé en ce que** la pellicule (1) est extensible.

4. Pansement adhésif selon une ou plusieurs des revendications précédentes, **caractérisé en ce que** le revêtement (2) sur un côté est un vernis protecteur.

5. Pansement adhésif selon une ou plusieurs des revendications précédentes, **caractérisé en ce que** le revêtement (2) sur un côté est un revêtement pulvérulent.

6. Pansement adhésif selon une ou plusieurs des revendications précédentes, **caractérisé en ce que** les revêtements (21, 22) sur les côtés opposés sont constitués chaque fois d'un matériau conducteur électrique.

7. Pansement adhésif selon la revendication précédente, **caractérisé en ce que** au moins un des matériaux conducteurs électriques est un métal précieux.

8. Pansement adhésif selon une ou plusieurs des revendications précédentes, **caractérisé en ce que** le support se compose d'un polymère.

9. Pansement adhésif selon la revendication précédente, **caractérisé en ce que** le support se compose de polyester.

10. Pansement adhésif selon une ou plusieurs des revendications précédentes, **caractérisé en ce que** le revêtement sur au moins un côté a une épaisseur de moins de 5 µm, de préférence moins de 500 nm.

11. Pansement adhésif selon une ou plusieurs des revendications précédentes, **caractérisé en ce que** le revêtement a été généré par métallisation sous vide d'argent et/ou d'or sur la pellicule (1).

12. Procédé de fabrication d'un pansement adhésif destiné à être appliqué sur la peau à des fins médicales, cosmétiques et/ou orthopédiques, en particulier pour la fabrication du pansement adhésif selon l'une quelconque des revendications précédentes, étant donné que
• une pellicule (1) en un matériau support est dotée sur un premier côté d'un revêtement en or (21) ou d'un revêtement métallique couleur or et
• la pellicule (1) est dotée sur un deuxième côté opposé d'un revêtement en argent (22) ou d'un revêtement métallique argenté de manière à ce que la pellicule (1), y compris les deux revêtements (21, 22), ait une épaisseur de moins de 100 µm,
étant donné que de la colle est prévue circonférentiellement sur les bords circonférentiels de la pellicule (1) et/ou hors des bords circonférentiels de la pellicule (1), de manière à ce que, lorsque le pansement adhésif est collé sur la peau d'un patient, la surface de la peau recouverte par la pellicule (1) soit étanche à l'air ambiant.

13. Procédé selon la revendication précédente, dans lequel les revêtements sont générés par réalisation d'un vernis protecteur sur la pellicule (1), réalisation d'un revêtement pulvérulent sur la pellicule (1) ou par métallisation sous vide d'argent et/ou d'or sur la pellicule (1).
